# EUROPEAN PATENT APPLICATION

(11) **EP 0 681 026 A1**
(43) Date of publication of application: **08.11.1995**
(21) Application number: 95201559.2
(22) Date of filing: 29.02.1988
(51) Int. Cl.: C12N 15/74, A61K 39/04, C12N 1/20

(54) **Recombinant mycobacterial vaccine**

(30) Priority: 02.03.1987 US 20451
(62) Divisional of application: 88903026.8
(71) Applicant: WHITEHEAD INSTITUTE FOR BIOMEDICAL RESEARCH, Cambridge, MA 02142 (US)
(72) Inventor: Bloom, Barry R., Hastings on Hudson, New York 10706 (US); Davis, Ronald W., Palo Alto, Cambridge 94301 (US); Jacobs, William R. Jr., Bronx, NY 10462 (US); Young, Richard A., Winchester, Massachusetts 01890 (US)
(74) Representative: Holdcroft, James Gerald, Dr.

(57) **Abstract**

Recombinant mycobacterial vaccine vehicles capable of expressing foreign DNA which encodes at least one protein antigen for at least one pathogen against which an immune response is desired. The vaccine vehicles are useful for administration to mammalian hosts for purposes of immunization. A shuttle vector which replicates as a plasmid in a bacterium and which replicates as a phage in a mycobacterium is also disclosed. The shuttle vector is useful for transferring genetic material between different genera of microorganisms.

## Description

### Funding

Work described herein was supported by funding from the World Health Organization, Albert Einstein College of Medicine, The Whitehead Institute for Biomedical Research, and the National Institutes of Health.

### Background

### Immunization

Immunity to a foreign antigen (e. g., a pathogen or toxin) can be provided by passive transfer or active induction. In the former case, antibodies against the foreign protein pathogen are injected into an individual, with the result that short-term protection is provided. In the latter case, injection of a harmless (innocuous) form of the pathogen, a component of the pathogen, or a modified form of the toxin (i. e., a toxoid) stimulates the individual's immune system, conferring long-term protection.

Active immunity can be induced, provided an individual's immune system is competent, by using an appropriate antigen to stimulate the immune system. For example, immunization (vaccination) with an innocuous or attenuated form of the pathogen in this manner results in an immediate immune response, as well as immunological "memory", thus conferring long-term protection as well. In general, vaccines include inactivated, nonpathogenic or attenuated forms of a pathogen or infectious agent, which include antigenic determinants of the pathogen and thus elicit an immune response. Similarly, toxins, which are antigenic substances produced by microorganisms, plants and animals, can be converted to toxoids; that is, they can be modified to destroy their toxic properties but retain their antigenicity and, as a result, their ability to stimulate production of antitoxin antibodies and produce active immunity. Such toxoids can be used for vaccines against the toxin.

In both cases--that involving stimulation of an immune response by administration of an altered form of an infectious pathogen and that involving administration of a toxoid--presently-available procedures are generally effective but side effects and deaths resulting from the vaccination are known to occur.

Safer vaccines are now being developed through application of better knowledge of the antigenic determinants of a pathogen and of genetic engineering/recombinant DNA techniques. For example, it is possible to make a polypeptide (e. g. by chemical synthesis or expression of DNA encoding the polypeptide of interest) which is a component (e. g., an antigenic determinant) of a protein antigen known to elicit an immune response. Administration of the polypeptide to a host is followed by an immune response by the host to the antigenic determinant. Use of such a polypeptide is not accompanied by the risk of infection which accompanies use of live or attenuated vaccines.

Immunization (administration of a vaccine) is a common and widespread procedure and the vaccine used can be essentially "any preparation intended for active immunological prophylaxis", including preparations of killed microbes of virulent strains, living microbes of attenuated strains, and microbial, fungal, plant, protozoal or metazoan derivatives or products. Stedman's Illustrated Medical Dictionary (24th edition), Williams & Wilkins, Baltimore, p. 1526 (1982). In many cases, vaccines must be administered more than once in order to induce effective protection; for example, known anti-toxin vaccines must be given in multiple doses.

Childhood vaccination is commonplace and generally successful in developed countries, where there is ready access to health services and multiple immunizations (e.g. immunization against multiple pathogens and serial or multiple immunizations against a single pathogen) are possible. In the developing world, vaccination is far less common and far more problematic. For example, only about 20 percent of the 100 million children born in the developing world each year are vaccinated against diphtheria, pertussis, tetanus, measles, poliomyelitis and tuberculosis. It is estimated that each year, 5 million children in the developing world die and another 5 million children are physically or mentally disabled by these diseases, which could be prevented if adequate immunization were possible. Availability of effective vaccines which can confer long-term immunity with a single administration would, of course, be valuable in both developed and developing countries.

Vaccination of adults is also helpful in preventing many diseases in adults and, as is the case with children, in developing countries may prove to be difficult to carry out, particularly if multiple immunizations are necessary. Diseases such as leprosy, malaria, tuberculosis, and poliomyelitis, among others, have a high incidence among adults in Africa, Asia and Latin America and are the causes of thousands of deaths annually.

Much effort has been expended in developing vaccines against major diseases and, recently, consideration has been given to recombinant vaccine vehicles (e. g., genetically engineered viruses) to express foreign genes. For example, recombinant vaccinia virus, in which viral antigens are inserted into vaccinia virus--has been developed. For example, hepatitis B genes, influenza virus genes or DNA encoding rabies virus antigen have been spliced into vaccinia virus DNA in efforts to make vaccines. Panicali, D. et. al., Proceedings of the National Academy of Sciences, USA, 80: 5364-5368 (1983) ; Orr, T., Genetic Engineering News, p. 17, (March 1985) ; Paoletti, E. and D. Panicali, U. S. Patent 4,603,112.

It is widely agreed, however, that such recombinant vaccinia virus would have at least two important drawbacks as a vaccine. First, there is a significant mortality and morbidity (1:100,000) associated with vaccinia virus, which is untreatable. Second, vaccination with recombinant vaccinia of individuals previously exposed to vaccinia virus has often failed to produce satisfactory immunization levels. Fenner, F., New Approaches to Vaccine Development, R. Bell and G. Torrigiani (ed.), Schwabe & Co., p. 187 (1984).

To date, vaccines have been developed which, although effective in many instances in inducing immunity against a given pathogen, must be administered more than once and may be unable to provide protection, on a long-term basis, against a pathogen. In addition, in many cases (e. g., leprosy, malaria, etc.), an effective vaccine has yet to be developed.

### Mycobacteria

Mycobacteria represent major pathogens of man and animals. For example, tuberculosis is generally caused in humans by Mycobacterium (M.) tuberculosis and in cattle by Mycobacterium (M.) bovis (which can be transmitted to humans and other animals, in whom it causes tuberculosis). Tuberculosis remains widespread and is an important public health problem, particularly in developing countries. It is estimated that there are approximately 10 million cases of tuberculosis world-wide, with an annual mortality of 3 million. Joint International Union Against Tuberculosis and World Health Organization Study Group, Tubercle, 63:157-169 (1982).

Leprosy, which is caused by M. leprae, afflicts over 10 million people, primarily in developing countries. Bloom, B. R. and T. Godal, Review of Infectious Diseases, 5:657-679(1984). M. tuberculosis and mycobacteria of the avium-intracellulare-scrofulaceum (MAIS) group represent major opportunistic pathogens of patients with acquired immunodeficiency disease(AIDS). Centers for Disease Control, Morbidity and Mortality Weekly Report, 34:774 (1986). M. pseudotuberculosis is a major pathogen of cattle.

On the other hand, Bacille Calmette-Guerin (BCG), an avirulent strain of M. bovis, is the most widely used human vaccine in the world and has been used as a live vaccine for more than 50 years. In the past 35 years, it has been administered to over 5 billion people, with remarkably few adverse effects (e. g., estimated mortality of 60/billion). BCG has been found in numerous studies to have protective efficacy against tuberculosis. Recently, however, it was found not to be effective in preventing pulmonary tuberculosis in Southern India. Tuberculosis Prevention Trial, Madras, Indian Journal of Medical Research, 72 (suppl.):1-74 (1980).

Thus, although there are numerous vaccines available, including BCG, many are limited side value because they induce a limited immune response, must be given in multiple doses and/or have adverse side effects. In other cases (e. g., leprosy, malaria), a vaccine is simply unavailable. It would be of great value if a vaccine against a pathogen or pathogens of concern were available which provided long-term stimulation of immunity in recipients sufficient to provide protection against the pathogen(s) without adverse effects.

### Disclosure of the Invention

The present invention relates to genetically recombinant (genetically engineered) cultivable mycobacteria which express DNA of interest incorporated into them using genetic engineering techniques, and to methods of introducing DNA into mycobacteria to produce genetically recombinant mycobacteria. The resulting recombinant mycobacteria are particularly useful as vehicles by which the DNA of interest can be expressed, for example as vaccine vehicles which express protective antigens (i. e., antigens capable of eliciting an immune response in a host), such as those for one or more pathogens of interest or those useful in producing an anti-fertility vaccine vehicle. Pathogens of interest include any virus, microorganism, or other organism or substance (e. g., a toxin or toxoid) which causes disease.

The present invention also relates to methods of vaccinating a host with the recombinant mycobacterium to elicit protective immunity in the host. It further relates to a method of transferring genetic material between different genera of microorganisms and to a genetically engineered vector, referred to as a shuttle phasmid, useful for the transfer of genetic material between different genera of microorganisms. The shuttle phasmid replicates in bacteria (e.g., E. coli) as a plasmid and in mycobacteria as a phage. It has made it possible to introduce into mycobacteria, such as Mycobacterium smegmatis (M. smegmatis) and Mycobacterium bovis-BCG (BCG), DNA from another source (e.g., a source other than M. smegmatis or BCG). In addition, the present invention relates to use of the antigens expressed by the recombinant cultivable mycobacterium as vaccines or for diagnostic reagents.

In particular, the present invention relates to a vaccine vehicle which is genetically recombinant BCG or genetically recombinant M. smegmatis which expresses DNA, from a source other than the vaccine vehicle (referred to as foreign DNA), which encodes antigens for one or more pathogens of interest (any virus, microorganism or other organism or substance which causes disease). The vaccine vehicle of the present invention also expresses DNA encoding antigens useful in preventing conception (e.g., in vaccines useful as birth control agents.)

The pathogens for which protective antigens can be expressed by the recombinant vaccine include, but are not limited to, Mycobacterium leprae, Mycobacterium tuberculosis, Mycobacterium intracellulare, Mycobacterium africanum, Mycobacterium avium, malaria sporozoites and merozoites, diphtheria toxoid, tetanus toxoids, Leishmania, Salmonella, some Treponema, pertussis toxin, and other antigenic determinants, viruses (including measles, mumps, herpes, influenza, Schistosoma, Shigella, Neisseria, Borrelia, rabies, polio virus, human immunodeficiency virus (HIV), HTLV-I, HTLV-II and snake and insect venoms.

In one embodiment of the present invention, recombinant mycobacteria capable of expressing foreign DNA encoding such protective antigens are produced by integrating into the mycobacterial genome a gene or genes encoding protective antigen(s) of interest. A gene encoding a single antigen or two or more genes, each encoding an antigen, is/are inserted into a region of the mycobacterial genome which is nonessential for replication of the mycobacterium. It is also possible to insert one or more genes, each encoding an antigen of interest, into a temperate bacteriophage which, after introduction into a mycobacterium, can integrate into the mycobacterial chromosome and subsequently be expressed with the chromosomal DNA. Alternatively, a gene or genes encoding antigen(s) of interest can be introduced into a mycobacterium in such a manner that expression of the gene(s) occurs extrachromosomally (e.g., episomally). For example, a gene or gene of interest is/are cloned into a mycobacterial plasmid and introduced into a cultivable mycobacterium, where it undergoes episomal replication (extrachromosomal replication).

The vaccine of the subject invention has important advantages over presently-available vaccines. First, mycobacteria have adjuvant properties among the best currently known and, thus, stimulate a recipient's immune system to respond to other antigens with great effectiveness. This is a particularly valuable aspect of the vaccine because it induces cell-mediated immunity and will, thus, be especially useful in providing immunity against pathogens in cases where cell-mediated immunity appears to be critical for resistance. Second, the mycobacterium stimulates long-term memory or immunity. As a result, a single (one-time) inoculation can be used to produce long-term sensitization to protein antigens. That is, a single innoculation can result in sensitization lasting 5 to 50 years. Using the vaccine vehicle of the present invention, it is possible to prime long-lasting T cell memory, which stimulates secondary antibody responses neutralizing to the infectious agent or the toxin. This is useful, for example, against tetanus and diphtheria toxins, pertusis, malaria, influenza, herpes viruses and snake venoms.

BCG in particular has important advantages as a vaccine vehicle in that: 1) it is the only childhood vaccine currently given at birth; 2) in the past 40 years, it has had a very low incidence of adverse effects, when given as a vaccine against tuberculosis; and 3) it can be used repeatedly in an individual (e. g., in multiple forms).

A further advantage of BCG in particular, as well as mycobacteria in general, is the large size of its genome (approximately 4 x 10⁶ bp in length). Because the genome is large, it is able to accommodate a large amount of new (i. e., foreign) DNA and, thus, can be used to make a multi-vaccine vehicle (i. e., one carrying foreign DNA encoding protective antigens for more than one pathogen).

The genetically engineered vector of the present invention is unique in its use for the transfer of genetic material between different genera of microorganisms. Specifically, the vector is comprised of DNA from a bacterial plasmid, genetically spliced into a mycobacteriophage. The resulting recombinant shuttle vector includes a truncated mycobacteriophage DNA molecule into which an E. coli cosmid (an E. coli plasmid containing the lambda cohesive end sites (COS)) has been cloned. It is referred to as a phasmid, because of its ability to replicate in E. coli as a plasmid (where it expresses drug, e. g., ampicillin, resistance) and in mycobacteria as a phage. For example, DNA of a plasmid from E. coli is genetically spliced into the mycobacteriophage TM4, resulting in production of the shuttle phasmid phAE1. Use of the shuttle plasmid makes it possible to manipulate genetic material in E. coli an then transfer it efficiently into cultivable mycobacteria (e. g., BCG, M. smegmatis). Thus, for the first time, it is possible to introduce foreign DNA by infection into mycobacteria.

By introducing a gene encoding a pathogen of interest or more than one gene encoding more than one pathogen of interest into mycobacteria, such as BCG and M. smegmatis, in which they are expressed, it is possible to make vaccines which, unlike presently-available vaccines, can provide long-term immunity against one or more pathogens. The long-term immunity can be conferred with one innoculation and with a very low incidence of adverse effects in those innoculated.

### Brief Description of the Drawings

Figure 1 shows results of transfection of Mycobacterium smegmatis spheroplasts with mycobacteriophage D29 DNA.

Figure 2 is a schematic representation of the construction of the shuttle phasmid, phAE1.

Figure 3 shows an ethidium bromide stained 0.7% agarose gel of mycobacteriophage TM4 DNA and shuttle phasmid phAE1 DNAs digested with KpnI (panel A) and a Southern blot analysis of phasmid phAE1 using pHC79 as a probe (panel B).

In panel A, lane 1 contains lambda DNA digested with Hind III; lanes 2 and 3 contain TM4 DNA that was unligated (lane 2) or ligated (lane 3) prior to digestion cut with KpnI; lanes 4 and 5 contain phAEI DNA isolated from phage particles propagated on M. smegmatis (lane 4) and phAE1 isolated from E.coli cells as a plasmid (lane 5). Note that the arrows point to the 2.1Kb and the 1.8Kb fragments that form a 3.9Kb fragment when ligated at the cohesive ends.

In panel B, the autoradiograph of Panel A is shown after blotting onto a Biotrans nylon membrane (ICN) and probing with pHC79 DNA that had been nick-translated with ³²P-dCTP.

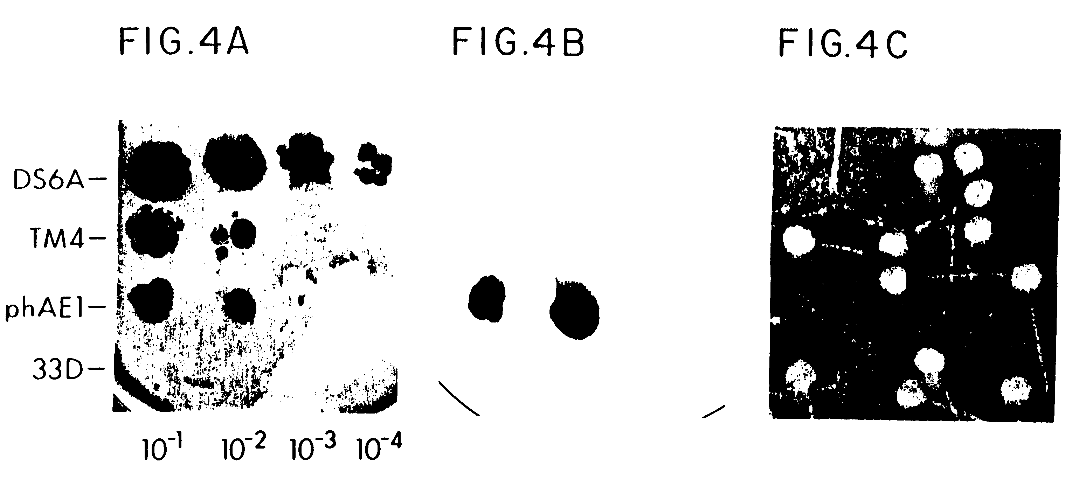

Figure 4 shows replication of phAE1 on BCG. It compares lysis of the Glaxo vaccine strain of BCG by DS6A, which is a mycobacteriophage known to plaque on M. tuberculosis and BCG, but not on other mycobacteria; phage 33D, known to plaque on M. smegmatis and not BCG; and phage TM4, which plaques on both species.

Figure 4A shows lysis of BCG by the phages. Titres of phage (pfu/ml) used at 10⁻¹ dilution were: DS6a, 2 x 10⁶ on M. tuberculosis, H37Ra, 33D, 2 x 10⁶ on M. smegmatis, mc²6; TM4, 3 x 10⁸ on mc₂6; and phAE1, 3 x 10⁸ on mc²6. Dilutions of phages (5ul) were spotted on a soft agar overlay containing 10⁸ BCG cells. Resulting lysis was photographed after incubation for 10 days at 37°C.

Figure 4B shows the present of cosmid DNA in phAE1. Plaque lifts on these plates were carried out as described below and hybridized with ³²P-labelled pHC79 DNA; this was followed by autoradiography.

Figure 4C is an electron micrograph of shuttle phasmid phAE1 phage particles. Phage particles that had been purified on CsCl gradients were placed on carbon coated, Parloidon-coated grids, blotted and washed with one drop of 1% phosphotungstic acid. Electron micrographs were taken using a JEOL 1200EX electron microscope at 80 kV, 30,000X.

### Detailed Description of the Invention

Mycobacterium bovis-BCG (BCG) is an avirulent M. bovis derivative which is widely used throughout the world and is commonly used to provide protection against tuberculosis, although its effectiveness has recently been called into question. Mycobacterium smegmatis is a nonpathogenic bacillus which shares antigenic and adjuvant properties with BCG. Both are also reasonably easy to grow in culture.

Because both mycobacteria have excellent adjuvant activity for induction of cell-mediated immunity, stimulate long-term memory (immunity) and have a low mortality associated with their use, they are excellent candidates as recombinant vaccines. That is, they are excellent candidates for use as vehicles (vaccine vehicles) into which genetic material of interest (DNA encoding one or more pathogens of interest against which an immune response is sought) can be inserted and subsequently expressed. Such DNA, which can be all or a portion of a gene or genes and which is incorporated into a mycobacterium, is referred to herein as foreign DNA. Such vehicles can be used as vaccines to provide immunity against the pathogen whose antigen is encoded by the foreign DNA. They can also be used as an anti-fertility "vaccine" vehicle; for example, mycobacteria containing DNA encoding antigens such as human gonadotropic hormone (HGH) fragments can be used as an anti-fertility vaccine and administered as birth control agent. Foreign DNA is DNA from a source other than the mycobacterium into which the DNA is being incorporated (e.g., in the case of BCG, DNA from other than BCG). A pathogen is any virus, microorganism, or other organism or substance (e. g., toxins) which causes disease. Because of their large genomes (e.g., the BCG genome is about 4x10⁶ bp long), mycobacteria can accommodate large amounts of new (foreign) DNA and, thus, can serve as multi-vaccine vehicles.

Until the present time, however, it has not been possible to transform a mycobacterium through the use of plasmid DNA. This is partly due to the fact that knowledge of the molecular biology and genetics of mycobacteria is much less advanced than that of a great many genera of microorganisms, despite the fact that lysogeny (i.e., induction of general lysis in a culture of another strain, without lysis of the mycobacteria themselves) and transfection have been described in some species and a wide variety of mycobacteriophages exists.

A principal objective of work on the development of a recombinant mycobacterium to be used as a vaccine vechicle is the introduction into the mycobacterium of DNA vectors that direct the expression of genes whose products are important for protection against one or more pathogens. It is now possible, using the method and the shuttle vector of the present invention, to introduce foreign DNA into a cultivable mycobacterium; that foreign DNA comprises DNA encoding one or more antigen or antigens of interest. The shuttle vector of the present invention is unique in that it replicates as a plasmid in bacteria and as a phage in mycobacteria. In a particular embodiment, the shuttle vector, which is referred to as a cohesive phasmid, includes two species of specific cohesive end (or cos sites) : one for lambda phage, which functions in E.coli; and one for mycobacteria (e.g., the mycobacteriophage TM, which functions in mycobacteria). That is, it contains two sets of cohesive ends. Because it contains one set for lambda and cohesive mycobacteria, it can be incorporated into both. The presence of the lambda COS sequence also makes it possible to use the efficient technique of cosmid cloning, which utilizes the lambda in vitro packaging system for efficient cloning of large DNA molecules into E. coli. Further, the shuttle vector has a unique EcoRI site into which antigen-encoding DNA can be inserted. Thus, the vectors have made it possible to develop a transfection system which permits introduction of recombinant DNA molecules into mycobacteria.

There are several means by which genetic material of interest can be incorporated into mycobacteria to produce recombinant mycobacteria of the present invention. For example, DNA of interest can be stably introduced into mycobacterial cells by cloning into a shuttle phasmid, particularly a temperate shuttle phasmid (e.g., a phage capable of lysogenizing a cell). Introduction of DNA of interest (foreign DNA) in this manner results in integration of the DNA into the mycobacterial chromosome. Alternatively, a plasmid vector can be used to introduce foreign DNA into mycobacteria, in which the DNA is expressed extrachromosomally.

It is also possible to introduce foreign DNA and cause it to integrate into host chromosomes without a phage. For example, this can be accomplished by homologous recombination, site specific recombination or nonhomologous recombination (e.g., by means of a transposon, which results in random insertion into host chromosomal material).

In order to successfully introduce foreign DNA (i.e., DNA encoding one or more antigens for one or more pathogens of interest) into a mycobacterium, such as BCG, by means of the shuttle vector of the present invention, the following general approach was followed. Although it is described in terms of M. smegmatis and BCG, it is to be understood that it can also be used to introduce foreign DNA into other mycobacteria and that these other mycobacteria containing foreign DNA can also be used as vaccine vehicles. In the case of slow growing mycobacteria (e.g., BCG and M. tuberculosis) to be used as vaccine vehicles, it is particularly valuable to go through (i.e., introduce DNA encoding an antigen or antigens of interest into) M. smegmatis and, subsequently, into BCG.

### Development of a shuttle vector to transfer DNA into mycobacteria

### Transfection of mycobacteriophage DNA into M. smegmatis

To develop a system that permits manipulation of DNA in mycobacteria, it was first necessary to develop an efficient means of transferring DNA into the bacillus. The technology used was a modification of that described by Okanishi and Hopwood in relation to the preparation of spheroplasts for Streptomyces. Streptomyces, like mycobacteria, are Actinomycetales. Okanishi, M. et al., Microbiology, 80: 389-400(1974); Hopwood, D.A. and H.M. Wright, Molecular Genetics, 162: 307-317 (1978). The modification of this technique for use with M. smegmatis was used in combination with the addition of polyethylene glycol to facilitate entry of DNA molecules into bacterial spheroplasts.

Because of the unavailability of useful selectable antibiotic resistance markers in plasmids for transforming mycobacteria, the system chosen to evaluate optimum conditions for DNA transfer into mycobacteria was the transfection of DNA from lytic mycobacteriophages. Two advantages of such a system are that results obtained were quantitative and readily visualized as plaques within 24 hours.

Transfection of mycobacteriophage DNA into M. smegmatis is described in detail in Example 1. Briefly, DNA was initially introduced into mycobacteria having all or a portion of the cell walls removed (i.e., protoplasts or spheroplasts), using polyethylene glycol. Transfection experiments were initiated with DNA from mycobacteriophage D29, which propagates on a wide variety of mycobacteria and forms large clear plaques on M. smegmatis. Plate lysates of D29 phage prepared on M. smegmatis consistently yielded greater than 10¹¹ pfu (plaque forming units) per ml of lysate. The harvested phages were twice purified on CsC1 equilibrium gradients; they banded at an equilibrium buoyant density of 1.51. Phage DNA was extracted by proteinase K treatment and phenol-chloroform extraction. Restriction analysis of ligated and unligated D29 DNA demonstrated that the phage genomic DNA was double stranded, 50 kb in size, and possessed cohesive ends.

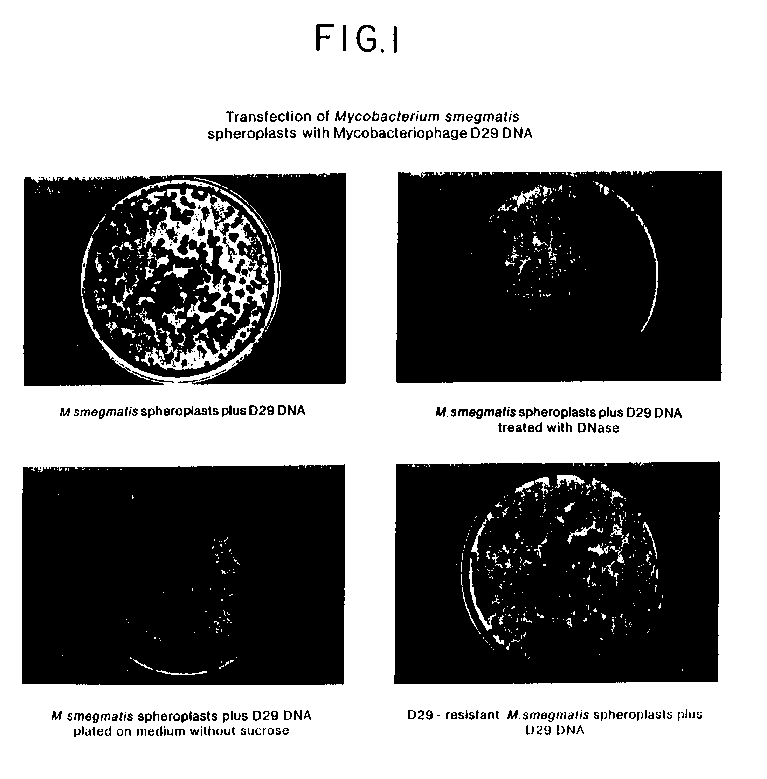

The results of transfection of M. smegmatis spheroplasts by mycobacteriophage D29 DNA are illustrated in Figure 1. Efficiencies of 10³ to 10⁴ pfu per ug D29 DNA were obtained, thus demonstrating the first efficient transfection system for mycobacteria. That these plaques were the result of transfection of M. smegmatis spheroplasts was demonstrated by the following: (i) transfection was abolished by DNase; (ii) osmotic shock of treated cells prevented productive transfection; and (iii) spheroplasts derived from a D29 phage-resistant mutant of M. smegmatis were transfected at frequencies comparable to the parent strain. Further refinement of these techniques made it possible to obtain frequencies greater than 10⁵ pfu per ug of D29 DNA.

### Introduction of foreign DNA into mycobacteria

An important step was the development of a vector that would permit both the manipulation and amplification of mycobacterial DNA constructs in E. coli, and subsequent transfer into and replication in mycobacteria. In particular, it was highly desirable to have the capability of introducing foreign DNA into fast-growing non-pathogenic mycobacterium (e.g., M. smegmatis), as well as into slow-growing mycobacteria (e.g., BCG and M. tuberculosis). Although plasmids have been found in some mycobacterial strains within the MAIS complex and in M. fortuitum, none have yet been described which replicate within M. smegmatis, BCG, or M. tuberculosis. With one exception, none of these plasmid possess selectable markers. Crawford, J.T. and J.H. Bates Infections and Immunity, 24: 979-981 (1979); Mizuguchi, Y. et al., Journal of Bacteriology, 146: 656-659 (1981) ; Meissner, P.S. and J.O. Falkinham, Journal of Bacteriology, 157: 669-672 (1984). In contrast, a variety of phages that replicate in M. smegmatis, BCG, and M. tuberculosis have been described and used for typing isolates.

The strategy used was to construct a vector which replicates as a plasmid in E. coli and as a phage in mycobacteria. One approach to accomplishing this development of a shuttle plasmid was based on the idea that since mycobacterial DNA is not expressed well in E. coli, it should be possible to clone, in a plasmid vector, a functional mycobacteriophage genome which would not lyse the E. coli host. It would, thus, be able to replicate in both types of organisms. Because transfection of M. smegmatis would yield mycobacteriophage particles, introduction of foreign DNA into the slow growing mycobacteria (e.g., BCG) could be achieved by phage infection. A bifunctional vector for Streptomyces has been described by Suarez and Chater. Suarez, J.E. and K.F. Chater, Nature, 286: 527-529 (1980). A lambda-ColE1 vector with dual properties in E.coli has been referred to by Brenner and co-workers as a phasmid. Brenner, S. et al., Gene, 17: 27-44 (1982).

For this purpose, the mycobacteriophage TM4 was used. TM4 has been reported to be a lysogenic phage isolated from M. avium. Timme, T. L. and P. J. Brennan, Journal of General Microbiology, 130: 205-209 (1984). It had been characterized as being a phage that lysogenizes M. smegmatis. It was shown to be capable of replicating in M. smegmatis, BCG, and M. tuberculosis and has been reported to be temperate.. This phage also has a double stranded DNA genome of 50 kb and possesses cohesive ends. It is possible, however, to use other mycobacteriophages having similar characteristics. The following procedures described as used with TM4 can also be used with such other mycobacteriophages in constructing a vector.

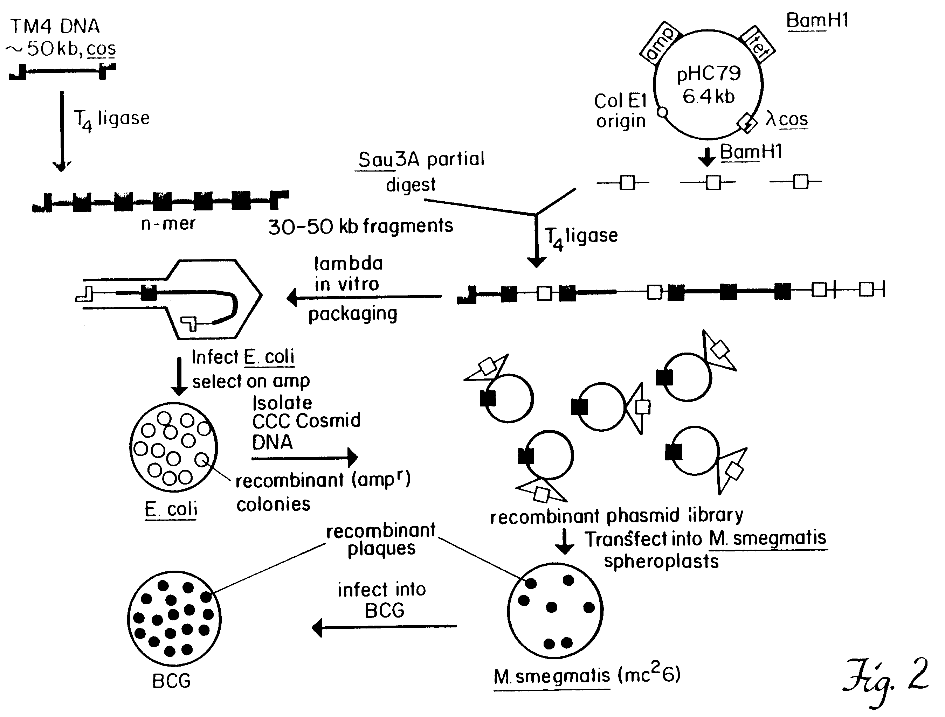

The strategy used to introduce an E.coli plasmid replicon into phage TM4 to generate a vector that replicates in E.coli as a plasmid and in mycobacteria as a phage is schematized in Figure 2. Plate stock lysates and genomic DNA of TM4 phage were prepared as described for D29 phage (see Example 1). TM4 DNA was ligated at high concentrations to form long concatamers and anneal the cohesive ends. The ligated DNA was partially digested with Sau3A. Sau3A cuts the TM4 genome frequently (e.g., an average of once every 300bp) to fragments 30-50 kb in size. It generates a set of DNA fragments whose lengths were that of the entire TM4 genome or TM4 genomes with small deletions, but are cleaved at any of the Sau3A sites within the genome. These DNA fragments were ligated to the 6.5 kb cosmid pHC79, which had been cleaved with BamHI. Hohn, B. and J. Collins, Gene, 9: 291-298 (1980). To select for recombinant molecules of the appropriate size, the ligation mixture was packaged into bacteriophage lambda heads in vitro. This selects for DNA fragments which contain lambda COS sites and are between 38 and 53 kb in size. The resulting phage particles were transduced into E. coli and colonies containing pHC79::TM4 DNA molecules were selected on media containing ampicillin. Plasmid covalently closed circular DNA was isolated from 40,000 pooled ampicillin-resistant (amp^{r}) colonies. Birnboim, H. and Doly, Journal of Nucleic Acid Research, 7: 1513-1525 (1979).

This library contains recombinant molecules of TM4 genomes into which pHC79 cosmid DNA had been randomly inserted in Sau3A sites around the TM4 genome. It was transfected into M. smegmatis spheroplasts to select for TM4 phages which had pHC79 inserted in non-essential regions. Such phages were, thus, shuttle phasmids. The transfection yielded 100 plaque forming units (pfu) per ug of plasmid DNA. Plaque lifts were used to screen for hybridization to ³²P-labelled pHC79 DNA; only 10 of 4000 plaques hybridized to the labelled pHC79.

Following plaque purification and propagation on M. smegmatis cells, one such phage was studied in detail and designated as phasmid, phAE1. Phasmid phAE1 was deposited (February 26, 1986) under Deposit No. 40306, at the American Type Culture Collection (Rockville, MD.) DNA was isolated from phAE1 phage particles grown on M. smegmatis,purified on CsC1 gradients, ligated to form concatamers, and packaged in vitro into bacteriophage lambda heads. The resulting particles transferred ampicillin resistance to E. coli cells and, when transfected, produced plaques on M. smegmatis. This was proof that phAE1 functions as a shuttle vector.

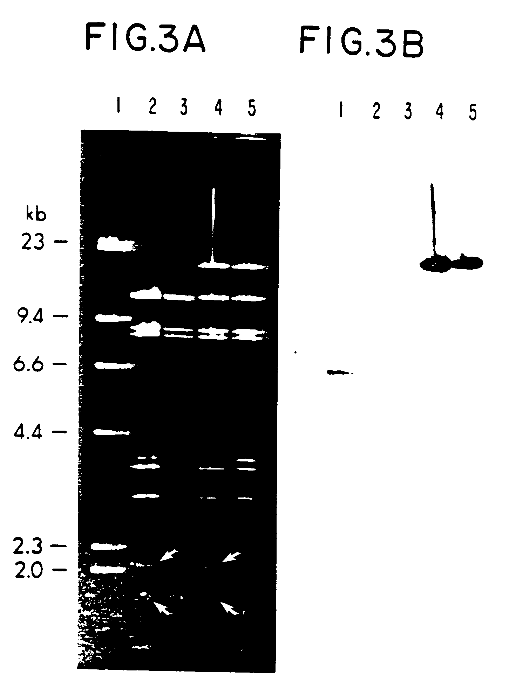

Restriction digests of phAE1 DNA isolated from phage particles propagated on M. smegmatis and of phAE1 DNA isolated as plasmid DNA isolated from E. coli showed identical patterns, except for the presence of unannealled fragments held together by the cohesive ends seen in the phage DNA preparation (Figure 3A). Southern analysis demonstrated that the cosmid pHC79 was cloned within one of the two 11 kb KpnI restriction fragments of the TM4 genome (Figure 3B). By electron microscopy, the phAE1 particles resemble bacteriophage lambda with hexagonal heads that average 50 um in diameter. However, these particles have long tails (180 to 220 um in length) with a disc-like baseplate present on many of the tails (Figure 4C). The structure is very similar to that of the parent TM4 phage. Timme, T. L. and P. J. Brennan, Journal of Gen. Microbiology, 130: 205-209 (1984).

Restriction analysis of DNAs from isolated phages resulting from the transfection of the pHC79::TM4 library into M.smegmatis that did not hybridize to pHC79 showed them to be identical. The phage appears to have resulted from a recombination event which occurred in transfected cells containing two or more pHC79::TM4 molecules, yielding a wild-type TM4 genome.

Of particular interest is the observation that the shuttle phasmid, phAE1, which was obtained from M. smegmatis, is like its parent TM4 in that it is able to infect and replicate in three different M. bovis-BCG vaccine strains tested: the Glaxo, Pasteur, and Danish BCGs. These results are presented in Figures 4A and 4B.

Thus, this demonstrates successful construction of E. coli mycobacterial shuttle phasmids that are recombinant DNA molecules that not only have the ability to replicate in E. coli as plasmids and in mycobacteria as a phages, but also have the ability to be packaged into bacteriophage lambda heads or into mycobacteriophage particles. It also demonstrates that recombinant DNA has been introduced into both a fast-growing mycobacterium (M. smegmatis) and a slow-growing mycobacterium (M. bovis-BCG). This makes it possible to infect BCG vaccine strains with the shuttle phasmids and, thus, to introduce cloned genes into mycobacteria. Thus, this eliminates the need to develop a transfection system for BCG. That is, because the E. coli-mycobacterial shuttle phasmid, upon transfection into mycobacteria is packaged into mycobacterial particles, foreign DNA can be introduced into slow-growing mycobacteria (e.g., BCG) by transduction, rather than transfection. Until now, this could not be done and it makes it possible to produce recombinant mycobacterial vaccine vehicles, which can be used to immunize against one or more antigens of interest.

The use of in vitro packaging to construct these phasmids can be extended as an efficient strategy for cloning of genes (e. g., genes, or foreign DNA, encoding an antigen or antigens for one or more pathogens against which an immune response is desired) into these vectors, as long as the size limits of the packaging system are not exceeded. It is also possible, by screening additional TM4::pHC79 recombinant phasmids, to determine the maximum amount of DNA that can be deleted from the TM4 phage and to define additional non-essential regions of the phage genome into which DNA can be inserted.

Introduction of new genes (e. g., foreign DNA encoding antigens) into mycobacteria by means of the shuttle phasmid entails cloning DNA fragments into the shuttle phasmid in E. coli and subsequently transfecting them into M. smegmatis spheroplasts. This yields recombinant phage particles containing the cloned gene(s). Using the resulting M. smegmatis spheroplasts containing the recombinant phages, it is possible to infect BCG with high efficiency (approaching 100% efficiency), thus introducing foreign DNA included in the recombinant phages into BCG. Development of conditions for establishing lysogeny or recombination, to permit stable expression of the foreign gene(s) in mycobacterial cells, is highly desirable.

### Introduction of foreign DNA into mycobacterial cells

The shuttle vector (phasmid) described above can be used to introduce foreign DNA which encodes one or more antigens for one or more pathogens of interest into mycobacteria, such as BCG or M. smegmatis. It can also be used, by introducing DNA encoding appropriate antigens, such as human gonadotropin hormone (HGH) fragments, into mycobacteria, to produce an anti-fertility "vaccine." The shuttle phasmid provides a means of accomplishing what has, until now, not been possible: manipulation and amplification of recombinant DNA constructs in a bacterium (e. g., E. coli, Streptomyces, Bacillus), or other organism (e.g., yeast) followed by transfer into and replication in a mycobacterium.

As a result, it is possible to produce recombinant mycobacterial vaccines which can be used to immunize individuals against, for example, leprosy, tuberculosis, malaria, diphtheria, tetanus, leishmania, salmonella, schistomiasis, measles, mumps, herpes, and influenza. Genes encoding one or more protective antigens for one or more of the disease-causing pathogens can be introduced into the mycobacterium. Of particular value is the ability to introduce genes encoding antigens of pathogens which require T-cell memory or effector function. Administration of the resulting recombinant mycobacterial vaccine to a host results in stimulation of the host's immune system to produce a protective immune response.

It is also possible, by homologous recombination, to exchange one gene for another gene and make insertional mutations in the genes for virulence of the mycobacterium, replacing gene(s) necessary for virulence with gene(s) whose presence results in a nonvirulent organism. In this way, it is possible to retain the genes encoding antigenicity, while removing those encoding or responsible for virulence of the organism.

A vaccine against a pathogen or toxin can be produced by the following procedure: DNA encoding an antigen (or antigens) for the pathogen or toxin against which protection is desired is obtained. The DNA can be obtained by isolation of the naturally-occurring DNA (e.g., from the pathogenic organism or toxin-producing organism); by cloning and amplification of the DNA sequence of interest, using known genetic engineering techniques (See, for example, Maniatis, T. et. al. Molecular Cloning: A Laboratory Manual, Cold Spring Harbor, N.Y. (1982).); or by mechanical synthesis.

By the following procedure, the gene or genes of interest (i.e., which encode one or more antigens against which immunity is desired) are cloned into the shuttle phasmid. This can be explained with reference to Figure 2, which is a schematic representation of the shuttle phasmid phAE1. The cohesive ends of the shuttle phasmid are ligated, using known techniques. The resulting shuttle phasmid material is cut (digested) with a unique restriction enzyme (e.g., a restriction enzyme which cuts at unique sites, such as EcoRI and EcoRV, in the shuttle phasmid). An alternative approach to cuts made in this way is the addition (by ligation) of a polylinker (oligonucleotide sequence) which is useful with (can be cut by) other restriction enzymes. In this case, the linker is cut open with a selected restriction enzyme, producing sites at which foreign DNA can be inserted.

In the first case (cutting using a unique restriction enzyme), the result is shuttle phasmid molecules which have been cut once and into which foreign DNA can be inserted. In the second case, there is also at least one site at which DNA can be inserted. An antibiotic-resistance-encoding gene (e.g., an ampicillin-resistance-encoding gene) and DNA encoding one or more antigens, against which immunity is desired, can be ligated, using known techniques, at the restriction sties. The DNA being inserted and the shuttle phasmid DNA are generally ligated in equal molar amounts.

The resulting ligated DNA, which in this case includes the shuttle phasmid DNA, an antibiotic resistance gene and antigen-encoding DNA, is packaged into bacteriophage lambda heads using lambda in vitro packaging mix. E.coli is subsequently transduced with the phage, with the result that it is possible to screen (using antibiotic-containing medium) for colonies containing the antibiotic-resistance-encoding gene and the antigen-encoding DNA.

The resulting "library" is transfected (infected) into M. smegmatis spheroplasts to select for plaques which contain shuttle phasmids containing cloned insert DNA. Subsequently, the recombinant M. smegmatis spheroplasts can be used to infect a cultivable mycobacterium, such as BCG, with high efficiency. As a result, the antigen-encoding DNA is introduced into mycobacterial genomic DNA, where it will be expressed.

Selection of BCG containing the DNA encoding one or more antigens integrated into their genomic DNA can be carried out using a selectable marker. One approach to selection of BCG containing DNA encoding one or more antigens, introduced by infection with the recombinant phage, is based on use of a selectable marker, which is an antibiotic resistance gene. In this case, the phasmid includes a gene encoding, for example, kanamycin resistance, viomycin resistance, thiostrepton resistance, hygromycin resistance, or bleomycin resistance.

A second approach in which a selectable marker is used to select BCG containing the DNA of interest is an auxotrophy strategy (i.e., one which relies on use of a mutant microorganism which requires some nutrient or substance not required by the organism from which the mutant was derived). In this case, a mycobacterium having the mutation is used and the gene which encodes the missing or mutated function is incorporated into the shuttle phasmid (which also contains antigen-encoding DNA). Selection for mycobacteria containing the antigen-encoding DNA is thus based on the ability of mycobacteria into which the shtutle phasmid is successfully introduced to survive, when grown on appropriate media.

For example, a system which includes a host mutant (e. g., M. smegmatis, BCG) and a selectable marker that complements the mutation can be used. Such a system can include a host mutant which is a pyrF⁻ BCG mutant and a selectable marker, such as a pyrF⁺ gene, present in the phasmid shuttle vector used to introduce the antigen-encoding DNA into the (mutant) BCG. In this case, the phasmid includes, in addition to the antigen-encoding DNA inserted into cosmid DNA, the pyrF⁺ gene. Thus, BCG mutants into which the phasmid is introduced by infection can be selected by plating on minimal media. An alternative approach is to use 2-deoxyglucose-resistant mutants; in this case, the mycobacterial glucokinase gene is cloned into the phasmid and is used for selection, as described above for pyrF.

Selection on this basis will result in BCG having the antigen-encoding DNA stably integrated into genomic DNA and expressed by the bacillus. For this, gene expression signals (e. g., promoters, ribosome binding sites) are included upstream of the foreign (antigen-encoding) DNA, to enable BCG containing the antigen-encoding DNA (modified BCG) to express it at levels sufficient to induce an immune response in a host to whom the modified BCG is administered.

It is also possible to select BCG containing DNA encoding one or more antigens by use of monoclonal antibodies. In this case, a gene or gene fragment encoding one or more epitopes of an antigen ( e. g., M. leprae or M. tuberculosis) for which monoclonal antibodies are available is introduced into the mycobacteria. Such monoclonal antibodies are used to select for recombinant BCG containing a gene or genes encoding one or more of these epitopes. The antigen genes introduced in this way contain a promoter sequence and other regulatory sequences. As a result, additional series (e. g., encoding other antigens) can be added, using genetic engineering techniques, in frame, such that recombinant BCG identified by monoclonal antibodies to one antigen would also be expressing other foreign antigen-encoding DNA so introduced.

A parallel strategy which makes use of a plasmid to introduce antigen-encoding DNA into cultivable mycobacteria and results in episomal (i.e., extrachromosomal)expression of the DNA can also be used to make a vaccine vehicle.

In this case, a selectable marker, which would make it possible to select cells containing the antigen-encoding DNA, is needed. The selectable marker can be, for example, an antibiotic-resistance-encoding gene or a gene which complements that missing in an auxotrophic mutant. For example, a gene encoding kanamycin resistance, viomycin resistance, thiostrepton resistance, nigromycin resistance or bleomycin resistance can be incorporated into the plasmid. In the auxotrophy strategy, an auxotrophic mycobacterial mutant (e.g., a pyr⁻F) mutant is isolated and the gene present in the corresponding wild-type (nonmutant) mycobacterium is incorporated into the plasmid. In addition to the pyr⁻F mutant, it is possible to isolate deoxyglucose mutants, which have a defect in the glucokinase gene, as well as others having mutations in other biosynthetic pathways (e.g., mutations in amino acid biosynthesis, vitamin biosynthesis and carbohydrate metabolism, such as arabinose and galactose).

In either approach, a mycobacterial mutant is selected and the gene which complements the mutation is incorporated into the plasmid vector, which also contains the antigen-encoding DNA of interest. The mycobacterial mutants into which the antigen-encoding DNA is successfully introduced will be identifiable (can be selected) by culturing on appropriately-selected media (e.g., media containing the antibiotic against which resistance is conferred, media containing or lacking the nutrients involved in the biosynthetic pathway affected in the mutant used).

Another component of a plasmid useful in introducing antigen-encoding DNA into the recombinant mycobacteria vaccine vehicle is an autonomously replicating sequence (e.g., a replicon), whose presence is a key determinant in allowing the plasmid to replicate autonomously (extra-chromosomally). These sequences can include, for example, a plasmid replicon, segments of a mycobacteriophage or chromosomal replication origins.

The design of the shuttle phasmid phAE1 includes several of these factors. For example, introduction of the E. coli cosmid pHC79 into the mycobacteriophage TM4 made it possible to provide an E. coli plasmid replicon origin and a selectable ampicillin resistance gene, as well as the bacteriophage lambda cohesive (cos) sequences and a unique EcoRI site. There are no EcoRI sites within the TM4 phage; the unique EcoRI site within phAE1 can be used for introducing foreign gene(s) into the phasmid. As described in Example 4, a 1.6 kb EcoRI fragment encoding the aminoglycoside phosphotransferase (aph) gene from Tn903 has been cloned into phAE1 using this cosmid cloning strategy.

There are several useful approaches to efficiently introduce the antigen-encoding DNA into a cutivable mycobacterium, such as BCG or M. smegmatis, which is to be used as a vaccine vehicle. For the plasmid, which includes DNA encoding the antigen(s) of interest, a selectable marker and an autonomously replicating sequence, protoplast fusion can be used for efficient introduction into the mycobacterium. In this case, E.coli or streptomyces having a cloned plasmid is fused, using known techniques, with a mycobacterial spheroplast. Using this approach, it is possible to transfer the foreign (antigen-encoding) DNA into the mycobacterium. Alternatively, E.coli minicells, which contain plasmid DNA and essentially no chromosomal DNA, can be used in carrying out a minicell protoplast fusion.

If, in the alternative, foreign DNA can be moved efficiently into the mycobacterium, an autonomously replicating sequence is not necessary and, instead, the foreign (e.g., antigen-encoding) DNA can be integrated into the mycobacterial chromosomes. This can be accomplished, for example, using minicell protoplast fusion. In this case, a selectable marker for the mycobacterium, which can be an antibiotic-resistance gene or a chromosomal mutation, can be cloned into an E.coli cosmid. Also present in the E.coli cosmid will be DNA which allows efficient integration into the chromosome of foreign DNA. For example, in M. leprae, a repetitive sequence occurs which appears to be associated with recombination; analogous sequences can be identified in and isolated from BOG and M. smegmatis, incorporated into the E. coli cosmid (along with the selectable marker) and result in a high degree of recombination.

A gene or genes of interest (encoding one or more antigens) can be incorporated into the construct described (e.g., which includes an E. coli replicon, a segment of mycobacterial chromosomal DNA associated with recombination (a recombinogenic sequence) and two selectable markers-one serving as a marker in E.coli and the second serving as a marker in the mycobacterium). The gene(s) can then be integrated into mycobacterial chromosomal DNA, such as BCG or M. smegmatis chromosomal DNA. If the gene(s) or interest are integrated in this way into M. smegmatis, it/they can also be moved into BCG by means of a general transducing phage. In this case, it is preferable to include, in addition to the other consruct components, two recominogenic sequences: one from M. smegmatis and one from BCG.

### Construction of Vaccines

It is possible, using the present invention, to construct a recombinant mycobacterial vaccine vehicle useful for immunizing against leprosy. Because of the extraordinary adjuvant activity of mycobacteria, such as BCG, such a vaccine would be effective in producing cell-mediated immunity, particularly of a long-term or enduring nature. Genes encoding protein antigens of the leprosy parasite M. leprae have been isolated by Young and are described in detail in co-pending U. S. Patent Application Serial No. 892,095, filed July 31, 1986, the teachings of which are incorporated herein by reference. In particular, genes encoding five immunogenic protein antigens (i. e., antigens of molecular weight 65kD, 36kD, 28kD, 18kD and 12kD) have been isolated. In addition, 6 different epitopes encoded by the gene for the 65kD antigen have been defined. At least one of these epitopes has been shown to be unique to M. leprae; the other epitopes have been shown to be shared with the 65kD proteins of other mycobacteria.

Through use of the shuttle vector of the present invention, it is possible to introduce into BCG one or more of the genes encoding M. leprae protein antigens, using methods described above and in the following examples. For example, the gene encoding the 65kD M. leprae antigen can be introduced into BCG, stably integrated into its genomic DNA and expressed at levels sufficient to stimulate or induce an immune response in a host to which it is administered. In this way, it is possible to construct a vaccine which is close to ideal, in that it contains one or more protective antigens of M. leprae, does not have tolerogenic determinants and has an excellent adjuvant for inducing cell-mediated immunity.

In a similar fashion, it is possible to construct a vaccine, using the shuttle vector and the method of the present invention, to provide specific protection against tuberculosis. Such a vaccine is particularly attractive because of the recently reported finding, described above, that presently-used vaccines are proving to be neffective. Genes encoding immunogenic protein antigens of the tubercle bacillus M. tuberculosis have been isolated and are described in co-pending U. S. patent application Serial No. 010,007, entitled "Genes Encoding Protein Antigens of Mycobacterium Tuberculosis and Uses Therefor", by Robert N. Husson and Richard A. Young, filed February 2, 1987, and in the continuation-in-part application (Attorney's Docket No. WHI86-06A, filed by the Express Mail procedure February 10, 1988), entitled "Genes Encoding Protein Antigens of Mycobacterium Tuberculosis and Uses Therefor", by Robert N. Husson, Richard A. Young and Thomas M. Shinnick, the teachings of which are incorporated herein by reference.

In this case, a gene encoding an immunogenic protein antigen of M. tuberculosis is introduced into BCG by means of the shuttle vector, as described above. It is also possible to introduce more than one M. tuberculosis gene, each encoding a protein antigen, into BCG. For example, a gene encoding immunogenic M. tuberculosis antigens of molecular weight 12kD, 14kD, 19kD, 65kD and 71kD, or a combination of two or more of these genes, can be inserted into BCG, stably integrated into genomic DNA and expressed. The result is a vaccine which is specific for immunization against tuberculosis and which induces long-lived immunity against the bacillus.

It is also possible, using the method of the present invention, to construct a multipurpose or multifunctional vaccine (i. e., a single vaccine vehicle which contains and expresses foreign DNA which includes more than one gene, each gene encoding a protein antigen for a different pathogen or toxin). For example, it is possible to introduce into BCG, using the shuttle vector phasmid described, a gene encoding a protein antigen for M. leprae, a gene encoding a protein antigen for M. tuberculosis, a gene encoding a protein antigen for Leishmania, and a gene encoding a protein antigen for malaria. Administration of this multi-valent vaccine would result in stimulation of an immune response to each antigen and provide long-term protection against leprosy, tuberculosis, leishmaniasis, and malaria.

The present invention will now be illustrated by the following examples, which are not to be considered limiting in any way.

### Example 1 Transfection of M. smegmatis spheroplasts with mycobacteriophage D29 DNA

Spheroplasts of the M. smegmatis strain mc²6 were prepared according to the following method. mc²6 is a single colony isolate that is the predominant colony type isolated from the ATCC 607 M. smegmatis stock culture. It forms orange rough colonies on regeneration media. Hopwood, D. A. et. al., In: Genetic Manipulation of the Streptomyces-A Laboratory Manual, The John Innes Foundation, Norwich, England (1985).

Spheroplasts of M. smegmatis were prepared as for Streptomyces, using media for spheroplast preparation described by Udou et. al. for M. smegmatis. Udou, T. et al., Journal of Bacteriology, 151: 1035-1039 (1982). mc²6 cells were grown in 40 ml of tryptic soy broth containing 1% glucose and 0.2% Tween 80 in a 250-ml baffled-flask at 37°C with moderate shaking to an A₆₀₀ = 0.2, at which time a 20% glycine solution was added to a final concentration of 1%. The cells were incubated for an additional 16 hours and then harvested at room temperature by centrifuging at 5000 x g for 10 minutes. The pellet was washed twice with 10 ml of 10.3% sucrose and then resuspended in protoplast (P) buffer containing 2mg/ml lysozyme solution. After a 2-hour incubation at 37°C, 5 ml of P buffer was added and the spheroplasts were pelleted by centrifuging at 3000 x g for 7 min. The pellet was resuspended in 10 ml P buffer and used within 3 hours.

mc²-11 was isolated as a spontaneous D29-resistant isolate of the ATCC 607 M. smegmatis stock culture when 10⁸ cells were mixed with 3 x 10⁸ D29 plaque-forming units and plated on tryptic soy agar plates. D29-resistant colonies arose at a frequency of 10⁻⁷.

mc²6 were mixed with 1 ug of D29 DNA; one tenth of the resulting mixture was plated on tryptic soy agar plates, with or without 0.5M sucrose. They were then overlayed with the appropiate soft agar containing 10⁸ mc²6 cells. The DNase treatment was performed by adding DNase I (Sigma), at a final concentration of 50 ug/ml, to the D29 DNA.

Equivalent amounts of mc²11 spheroplasts were used in the same manner, but then subsequently overlayed with mc²6 cells to assay plaque forming units (pfu).

Phage Plate Stocks: Plate lysates of D29 were prepared on tryptic soy agar media containing 2mM CaCl₂. M. smegmatis cells that had been grown in a baffled flask at 37°C in Middlebrook 7H9 broth containing ADC enrichment to midlog phase were mixed with phage diluted in MP buffer (10mM Tris-HCl, pH 7.6 -10 mM MgCl₂-100 mM NaCl-2 mM CaCl₂) and incubated at 37°C for 36 hours, until plates were confluent. The phage were harvested with MP buffer and then purified on two CsCl equilibrium gradients, followed by extensive dialysis against MP buffer. DNA was extracted from phage by adding EDTA to a final concentration of 50 mM and treating with proteinase K at 100 ug/ml at 55°C for 24 hours, followed by phenol-chloroform extraction, and extensive dialysis against TE buffer.

Transfection: For each transfection, 2.5 ml of the spheroplast suspension was pelleted in a conical 15-ml polystyrene tube. The supernatant fluid was carefully decanted and the spheroplasts were resuspended in the remaining drop of buffer. After adding 1 ug of DNA in a total volume of less than 10 ul, 0.5 ml of a 25% PEG-1000 (J.T. Baker Chemical Co., Phila, PA) solution prepared in P buffer was added. The resulting combination was mixed. Within 3 min, 5 ml of P buffer was added to the mixture and the spheroplasts were pelleted as above. After carefully pouring off the supernatant fluid, the pellet was resuspended in 1 ml of P buffer and samples were transferred to tryptic soy agar with or without 0.5 M sucrose. The plates were then overlayed with 3.0 ml of soft tryptic soy agar and incubated at 37°C. The plaques were counted after 24 hours of incubation.

### Example 2 Construction of the shuttle phasmid phAE1

TM4 phage DNA was ligated at a concentration of 250 ug/ml. Aliquots were partially digested with Sau3A that was serially diluted; fragments that averaged 30 to 50 kb in length (as analyzed by agarose gel electrophoresis gel electrophoresis) were obtained in this manner. These fragments were ligated at a 1:2 molar ratio of TM4 fragments to pHC79 that had been cleaved with BamHI. The packaging of an aliquot of this ligation with in vitro packaging mix (Gigapack plus, Stratagene, San Diego, CA) and subsequent transduction into ER1381 (hsdR mcrA⁺ mcrB⁺, E. Raleigh), yielded 10⁶ ampicillin colonies per ug of TM4 DNA insert, when plated on L agar containing ampicillin at 50ug/ml.

A pool of 40,000 ampicillin-resistant clones was prepared by homogenizing colonies in L broth with a glass spreader. Plasmid was isolated from pools of clones by alkaline-SDS extraction, followed by phenol-chloroform extraction and concentration with ethanol. Covalently-closed plasmid DNA was transfected into mc²6 spheroplasts as described in Example 1. The plaques were screened for the presence of pHC79 by performing plaque lifts using the protocol of Benton and Davis and Biotrans nylon membranes (ICN). Benton, W. D. and R. W. Davis, Science, 196: 180-182 (1977). The membranes were hybridized with pHC79 DNA that had been nick-translated with ³²P-dCTP and autoradiography was performed.

### Example 3 Infection of BCG and M. smegmatis with shuttle plasmid phAE1

BCG-Glaxo (W. Jones) was propagated in Middlebrook 7H9 broth (Difco) containing ADC enrichment (Difco) and 0.5% Tween 80 (Sigma) in standing cultures at 37°C. Lawns of BCG-Glaxo or mc²6 cells were prepared by mixing 10⁸ BCG-cells with supplemented top soft agar and pouring on Dubos agar without Tween 80 (Gibco) supplemented with CADS enrichment (Difco). Jones, W.D., Jr., Tubercle, 60: 55-58 (1979). The 4 phages, DS6A, TM4, phAE1, and 33D were serially diluted and spotted on the two lawns. The plates wre read at 14 days and 2 days for BCG-Glaxo and M. smegmatis, respectively.

### Example 4 Cloning of aminoglycoside phosphotransferase gene into phAEI

A 1.6 kb EcoRI fragment encoding the aminoglycoside phosphotransferase gene (aph) from Tn903 was cloned into phAE1 by taking advantage of cosmid cloning strategy. Plasmid phAE1 DNA was isolated from E. coli and cut with EcoRI, the 1.6 kb fragment was ligated to these large DNA molecules. The ligation product was packaged into phage lambda in vitro, yielding particles which transduced kanamycin-resistance and ampicillin-resistance to E. coli cells. Plasmid DNA was isolated from these E. coli cells and shown to yield high frequencies of plaque-forming units when transfected into M. smegmatis mc²6 protoplasts. This demonstrates that it is possible to clone at least 1.6 kb of additional DNA into the unique EcoRI site of phAE1. Similar results were obtained with the shuttle phasmid phAE2, a shuttle vector which has similar characteristics to those of phAE1 but is 2 kb smaller in size than phAE1, which should allow for the cloning of at least 3.6 kb of additional DNA. In both cases, introduction of the aph gene resulted in introduction of a new NruI site, providing proof that additional DNA fragments can be cloned and stably maintained in the shuttle phasmids. Thus, these vectors without further modification can be useful for cloning additional genes into mycobacteria.

### Example 5 Stable expression of a selectable marker in mycobacteria using a shuttle phasmid.

Shuttle phasmids were constructed from the phage L1 (ATCC #27199) in a manner similar to those constructed for the TM4 phage. Doke, S., Kumamoto Medical Journal, 34:1360-1373(1960). All of the L1-shuttle phasmids identified have the ability to lysogenize M. smegmatis. L1 has been shown to integrate into M. smegmatis chromosomal material and to form stable lysogens. Other phage, such as L3 (ATCC #27200), a phage which remains as a plasmid (extrachromosomal) and L5 (ATCC #27201) can also be used in constructing shuttle phasmids. Results showed that these shuttle phasmids will lysogenize M. smegmatis and thus made it possible to stably integrate foreign DNA into mycobacteria for the first time. The aph gene was cloned into the unique EcoRI site of the L1-shuttle phasmid designated phAE15, as described above for the TM4-shuttle phasmids in E. coli. M. smegmatis cells (mc²6) were overlayed on top of agar on a Dubos agar plate containing kanamycin. Dilutions of the shuttle phasmid phAE15 and phAE19 (phAE15 with the clone aph gene) were spotted on the agar lawn. The plate was incubated 5 days at 37°C for 5 days. The colonies that grew all had been lysogenized with the L1-shuttle phasmid into which the aph gene had been cloned. The resulting shuttle phasmid, phAE19, was able to lysogenize M. smegmatis cells. The resulting lysogens expressed the cloned aph gene because they were resistant to kanamycin. Furthermore, these lysogens yielded mycobacteriophage particles that also expressed the kanamycin-resistant phenotype upon subsequent transfer and lysogenization of kanamycin-sensitive M. smegmatis cells. Transfer of these phages results in cotransduction of the lysogenic state (i.e. immunity to superinfection) and kanamycin resistance. The phage L1 phage, used to lysogenize M. smegmatis, does not plaque on BCG. However, variants of both L1 and the shuttle phasmid phAE19 which do form placques on BCG have been isolated. These can be tested for their ability to introduce and stably express foreign genes in BCG and M. tuberculosis by means of temperate shuttle phasmids. Thus, these phages have the ability to stably introduce foreign DNA into M. smegmatis. In addition, host range variants (e.g., phAE19) which will infect and lysogenize BCG have been isolated. This has made it possible to produce a recombinant mycobacterium, containing DNA of interest. Such recombinant mycobacteria can be used as a vaccine.

### Equivalents

Those skilled in the art will recognize, or be able to ascertain, using no more than routine experimentation, many equivalents to the specific embodiments of the invention described specifically herein. Such equivalents are intended to be encompassed in the scope of the following claims.

The invention covers a recombinant mycobacterium capable of expressing foreign DNA incorporated therein.

The foreign DNA may encode at least one protein antigen.

The foreign DNA may encode at least one protein antigen selected from the group consisting of:
a. Mycobacterium leprae antigens;
b. Mycobacterium tuberculosis antigens;
c. malaria sporozoites;
d. malaria merozoites;
e. diphtheria toxoid;
f. tetanus toxoids;
g. Leishmania antigens;
h. Salmonella antigens;
i. Mycobacterium africanum antigens;
j. Mycobacterium intracellulare antigens;
k. Mycobacterium avium antigens;
l. Treponema antigens;
m. Pertussis antigens;
n. Herpes virus antigens;
o. Measles virus antigens;
p. Mumps virus antigens;
q. Shigella antigens;
r. Neisseria antigens;
s. Borrelia antigens;
t. rabies antigens;
u. polio virus antigens;
v. Human immunodeficiency virus antigens;
w. snake venom antigens; and
x. insect venom antigens

The recombinant mycobacterium may be Mycobacterium bovis-BCG, Mycobacterium smegmatis or any genetic variants thereof.

The invention also embraces a recombinant mycobacterium which is recombinant Mycobacterium bovis-BCG,recombinant Mycobacterium smegmatis, or a genetic variant thereof, said recombinant mycobacterium capable of expressing foreign DNA encoding at least one protein antigen.

The antigen is preferably selected from the group consisting of:
a. Mycobacterium leprae antigens;
b. Mycobacterium tuberculosis antigens;
c. malaria sporozoites;
d. malaria merozoites;
e. diphtheria toxoid;
f. tetanus toxoids;
g. Leishmania antigens;
h. Salmonella antigens;
i. Mycobacterium africanum antigens;
j. Mycobacterium intracellulare antigens;
k. Mycobacterium avium antigens;
l. Treponema antigens;
m. Pertussis antigens;
n. Herpes virus antigens;
o. Measles virus antigens;
p. Mumps virus antigens;
q. Shigella antigens;
r. Neisseria antigens;
s. Borrelia antigens;
t. rabies antigens;
u. polio virus antigens;
v. Human immunodeficiency virus antigens;
w. snake venom antigens; and
x. insect venom antigens

The invention also covers a recombinant mycobacterium capable of expressing foreign DNA encoding at least one protein antigen, the foreign DNA stably integrated into mycobacterial genomic DNA.

The recombinant mycobacterium may be Mycobacterium bovis BCG, Mycobacterium smegmatis or a genetic variant thereof.

The invention also relates to a recombinant mycobacterium capable of episomal expression of foreign DNA encoding at least one protein antigen, the recombinant mycobacterium preferably being Mycobacterium bovis-BCG or Mycobacterium smegmatis.

Also contemplated is a shuttle vector which replicates as a plasmid in a bacterium and which replicates as a phage in a mycobacterium.

The shuttle vector of the invention is preferably for use with a bacteriun selected fran the group consisting of E. coli, Streptomyces, and Bacillus and the nycobacterium is Mycobacterium bovis-BCG, Mycobacterium smegmatis or a genetic variant thereof.

The invention also contemplates
a recombinant shuttle vector, comprising:
a. mycobacteriophage DNA; and
b. cosmid DNA,
the cosmid DNA inserted into a non-essential region of the mycobacteriophage DNA.

The mycobacteriophage DNA may be DNA from a temperate mycobacteriophage and the cosmid DNA is from an E. coli cosmid.

Also covered is a recombinant shuttle vector comprising two sets of cohesive end sites: a first set comprising mycobacteriophage cohesive end sites and a second set comprising E. coli cosmid cohesive end sites.

Also contemplated is a shuttle vector comprising mycobacterial genomic DNA; lambda coliphage DNA and E. coli plasmid DNA, said E. coli plasmid DNA including an origin of replication and at least one drug resistance marker.

In a particular embodiment, the invention relates to the shuttle phasmid phAE1, deposited at the American Type Culture Collection under Deposit No 40306.

Also embraced by the invention is a method of making an E. coli- mycobacterial shuttle phasmid, comprising inserting an E. coli cosmid into a non-essential region of mycobacteriophage genomic DNA.

Also covered is a method of introducing foreign DNA into a mycobacterium, comprising introducing infectious mycobacteriophage comprising a recombinant DNA molecule into the mycobacterium.

The invention also relates to a method of incorporating a gene encoding an antigen of interest into a mycobacterium, comprising the steps of:
a. ligating the cohesive ends of a shuttle phasmid;
b. cutting the shuttle phasmid at a unique site with a restriction enzyme;
c. ligating the cut shuttle phasmid produced in (b) with a gene encoding antibiotic resistance and a gene encoding the antigen of interest, to produce a shuttle phasmid which includes the gene encoding antibiotic resistance and the gene encoding the antigen of interest;
d. packaging the shuttle phasmid produced in (c) into bacteriophage lambda heads;
e. transducing E. coli with bacteriophage produced in (d) and culturing transduced E. coli on medium containing the antibiotic against which resistance is conferred by the presence of the gene encoding antibiotic resistance, to select colonies containing the gene encoding the antigen of interest;
f. infecting cultivable mycobacteria with the colonies selected in (e), under conditions appropriate for introduction of the gene encoding the antigen of interest into the mycobacterial genome and expression of the antigen of interest.

The invention also relates to a method of making a recombinant shuttle phasmid, comprising:
a. ligating mycobacteriophage genomic DNA comprising cohesive end sites, to produce concatamers and to anneal the cohesive end sites;
b. partially digesting concatamers produced in (a) with the restriction enzyme Sau3A, to produce fragments of the concatamers;
c. obtaining fragments 30-50kb in size;
d. cleaving bacterial plasmid DNA, comprising cohesive end sites, with the restriction enzyme BamHI;
e. ligating mycobacteriophage fragments obtained in (c) with cleaved bacterial plasmid DNA, under conditions appropriate for formation of recombinant molecules; and
f. selecting recombinant molecules comprising mycobacteriophage genomic DNA and bacterial plasmid DNA, the bacterial plasmid DNA inserted in a non-essential region of the mycobacteriophage genomic DNA.

Also contemplated is a vaccine comprising a recombinant cultivable mycobacterium which expresses foreign DNA encoding at least one protein antigen. Here, the recombinant cultivable mycobacterium may be Mycobacterium bovis-BCG, M. smegmatis or a genetic variant thereof, and the vaccine may additionally comprise an appropriate carrier.

The invention also relates to a method of immunizing a mammalian host against one or more pathogens, comprising administering to said host a recombinant mycobacterium, said recombinant mycobacterium having incorporated therein foreign DNA encoding at least one protein antigen for each of said pathogens.

Also contemplated is a method of making a vaccine for immunization of a mammalian host against one or more pathogens comprising introducing into Mycobacterium bovis-BCG foreign DNA encoding at least one protein antigen for each of said pathogens.

## Claims

1. A recombinant mycobacterium (e.g. Mycobacterium bovis-BCG, Mycobacterium smegmatis or any genetic variants thereof) which expresses heterologous DNA (e.g. encoding at least one antigen) which is stably integrated into mycobacterial genomic DNA, or a recombinant mycobacterium which is recombinant Mycobacterium bovis-BCG, recombinant Mycobacterium smegmatis, or a genetic variant thereof, which recombinant mycobacterium expresses heterologous DNA encoding at least one antigen, or a vaccine comprising a recombinant mycobacterium (e.g. recombinant Mycobacterium bovis-BCG, recombinant Mycobacterium smegmatis, or a genetic variant thereof) which expresses heterologous DNA encoding at least one antigen, further comprising e.g. an appropriate carrier.

2. The recombinant mycobacterium or vaccine of claim 1 wherein the heterologous DNA is stably integrated into mycobacterial genomic DNA or present in a plasmid (for example a bacterial-mycobacterial shuttle plasmid, e.g. a shuttle plasmid having an origin of replication functional in mycobacterium and an origin of replication function in E. coli) for episomal expression.

3. The recombinant mycobacterium of any one of the preceding claims wherein the antigen is derived from Mycobacterium leprae, Mycobacterium tuberculosis, malaria sporozoites or merozoites, diphtheria toxoid, tetanus toxoids, Leishmania, Salmonella, Mycobacterium africanum**,** Mycobacterium intracellulare, Mycobacterium avium, Treponema, Pertussis, herpes virus, measles virus, mumps virus, Shigella, Neisseria, Borrelia, rabies virus, polio virus, HIV virus, snake venom and insect venom.

4. A recombinant shuttle vector which replicates as a plasmid in a bacterium (e.g. E. coli, Streptomyces or Bacillus) and which replicates as a phage in a mycobacterium (e.g. Mycobacterium bovis-BCG, Mycobacterium smegmatis or a genetic variant thereof), or a recombinant E. coli-mycobacterial shuttle vector, comprising:
a. mycobacteriophage DNA (e.g. temperate mycobacteriophage DNA); and
b. E. coli cosmid DNA which includes a gene for antibiotic resistance, the E. coli cosmid DNA inserted into a non-essential region of the mycobacteriophage DNA, or a recombinant shuttle vector comprising two sets of cohesive end sites: a first set comprising mycobacteriophage cohesive end sites and a second set comprising E. coli cosmid cohesive end sites or a shuttle vector capable of replication in a bacterium and in a mycobacterium comprising mycobacteriophage DNA; lambda coliphage DNA and E. coli plasmid DNA, said E. coli plasmid DNA including an origin of replication and at least one drug resistance marker, or the shuttle plasmid phAE1, deposited at the American Type Culture Collection under Deposit No. 40306.

5. A method of making an E. coli-mycobacterial shuttle plasmid, comprising inserting an E. coli cosmid into a non-essential region of mycobacteriophage genomic DNA.

6. A method of introducing foreign DNA into a mycobacterium, comprising introducing infectious mycobacteriophage comprising a recombinant DNA molecule encoding substance of interest into the mycobacterium, thereby producing a mycobacterium capable of expressing the recombinant DNA molecule.

7. A method of incorporating a gene encoding an antigen of interest into a mycobacterium, comprising the steps of:
a. ligating the cohesive ends of a shuttle plasmid;
b. cutting the shuttle plasmid at a unique site with a restriction enzyme;
c. ligating the cut shuttle plasmid produced in (b) with a gene encoding antibiotic resistance and a gene encoding the antigen of interest, to produce a shuttle plasmid which includes the gene encoding antibiotic resistance and the gene encoding the antigen of interest;
d. packaging the shuttle plasmid produced in (c) into bacteriophage lambda heads;
e. transducing E. coli with bacteriophage produced in (d) and culturing transduced E. coli on medium containing the antibiotic against which resistance is conferred by the presence of the gene encoding antibiotic resistance, to select colonies containing the gene encoding the antigen of interest;
f. infecting cultivable mycobacteria with the colonies selected in (e), under conditions appropriate for introduction of the gene encoding the antigen of interest into the mycobacterial genome and expression of the antigen of interest.

8. A method of making a recombinant shuttle plasmid, comprising:
a. ligating mycobacteriophage genomic DNA comprising cohesive end sites, to produce concatamers and to anneal the cohesive end sites;
b. partially digesting concatamers produced in (a) with the restriction enzyme Sau3A, to produce fragments of the concatamers;
c. obtaining fragments 30-50kb in size;
d. cleaving bacterial plasmid DNA, comprising cohesive end sites, with the restriction enzyme BamHI;
e. ligating mycobacteriophage fragments obtained in (c) with cleaved bacterial plasmid DNA, under conditions appropriate for formation of recombinant molecules; and
f. selecting recombinant molecules comprising mycobacteriophage genomic DNA and bacterial plasmid DNA, the bacterial plasmid DNA inserted in a non-essential region of the mycobacteriophage genomic DNA.

9. Use of a recombinant mycobacterium for the manufacture of a vaccine for inducing an immune response in a mammalian host against one or more pathogens, wherein said recombinant mycobacterium has incorporated therein foreign DNA encoding at least one protein antigen for each of said one or more pathogens, the recombinant mycobacterium e.g. being according to any one of claims 1 to 3.

10. A method of making a vaccine for inducing an immune response in a mammalian host against one or more pathogens, comprising the step of introducing into a mycobacterium (e.g. Mycobacterium bovis-BCG, Mycobacterium smegmatis or a genetic variant thereof) heterologous DNA encoding at least one protein antigen for each of said one or more pathogens and combining the recombinant mycobacterium with a pharmaceutically acceptable carrier, the heterologous DNA being for example stably integrated into the mycobacterial genome or present in a plasmid capable of episomal expression.

11. The recombinant mycobacterium of any one of claims 1 to 3 for use in therapy.

12. A recombinant mycobacterium for use in inducing an immune response in an animal wherein said mycobacterium expresses heterologous DNA encoding at least one antigen, or a recombinant mycobacteriophage containing in its genome DNA encoding a molecule of interest and a transcriptional promoter which controls expression of said molecule of interest-encoding DNA, wherein said molecule of interest is expressed upon incubation of said mycobacteriophage with the mycobacterium for which said mycobacteriophage is specific, wherein for example the molecule of interest is a protein, a polypeptide, an enzyme or a marker molecule.

## Amended claims

### Amended claims in accordance with Rule 86(2) EPC.

13. A mycobacterial plasmid vector capable of stable extrachromosomal replication in a mycobacterium comprising;
(a) DNA of interest,
(b) a selectable marker, and
(c) an origin of replication functional in mycobacteria.

14. A vector according to claim 13 which is a bacterial (e.g. E. coli) - mycobacterial shuttle plasmid vector which for example further comprises an origin of replication functional in E. coli (e.g. ColE1).

15. A vector according to claim 13 or claim 14 wherein the selectable marker permits selection in mycobacteria, the vector for example further comprising a selectable marker for selection in another bacterium (e.g. E. coli).

16. A vector according to any one of claims 13-15 wherein the vector comprises;
(a) E. coli plasmid pIJ666 DNA including the neo gene from Tn5 and the P15A origin of replication from pACYC184 and the cat gene from pACYC184, and
(b) plasmid pAL5000 DNA.

17. A vector according to claim 13 which is plasmid pYUP1100, as present in ATCC deposit No. 67745.

18. A recombinant mycobacterium (e.g M. bovis-BCG or M. smegmatis) having stably incorporated therein the vector of any one of claims 13-17, e.g. for expressing the DNA of interest.

19. The mycobacterium of claim 18 wherein the DNA of interest for expression encodes at least one protein or polypeptide, e.g. being selected from antigens, enzymes, lymphokines, immunopotentiators and reporter molecules.

20. A process for producing the mycobacterium of claim 18 or claim 19 comprising the step of transforming a mycobacterium with the vector of any one of claims 13 to 17, for example by electroporation.
